(19) **Europäisches Patentamt**
**European Patent Office**
**Office européen des brevets**

(11) **EP 3 100 666 A1**

(12) **EUROPEAN PATENT APPLICATION**
published in accordance with Art. 153(4) EPC

(43) Date of publication:
**07.12.2016 Bulletin 2016/49**

(21) Application number: **15742847.5**

(22) Date of filing: **15.01.2015**

(51) Int Cl.:
**A61B 1/00** *(2006.01)*    **A61B 17/28** *(2006.01)*

(86) International application number:
**PCT/JP2015/050942**

(87) International publication number:
**WO 2015/115196 (06.08.2015 Gazette 2015/31)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **29.01.2014   JP 2014014398**

(71) Applicant: **Olympus Corporation
Tokyo 192-8507 (JP)**

(72) Inventors:
• **YAMANAKA, Noriaki**
**Hachioji-shi,**
**Tokyo 192-8507 (JP)**
• **KISHI, Kosuke**
**Hachioji-shi,**
**Tokyo 192-8507 (JP)**

(74) Representative: **Schicker, Silvia**
**Wuesthoff & Wuesthoff**
**Patentanwälte PartG mbB**
**Schweigerstraße 2**
**81541 München (DE)**

(54) **MEDICAL WIRE AND MEDICAL DEVICE**

(57)    A medical wire includes a main wire portion and a sub wire portion fixed to the main wire portion, the main wire portion is disposed in a longitudinal direction of the medical wire, and the medical wire has a first region in which a radial cross-sectional area is relatively small and a second region in which a radial cross-sectional area is larger than that of the first region.

## FIG. 1

**Description**

[Technical Field]

The present invention relates to a medical wire and more particularly to a medical wire that can be suitably applied to a treatment tool having a flexible insertion section and a medical device having the medical wire.
Priority is claimed on Japanese Patent Application No. 2014-014398, filed January 29, 2014, the content of which is incorporated herein by reference.

[Background Art]

Conventionally, medical devices having end effectors for observation and treatment at a distal end of a long insertion section have been widely used. In such medical devices, a driving force may be transmitted to the distal end to curve the insertion section and to drive the end effectors. In general, a single wire or a stranded wire formed of metal wire is used as a transmission member for transmitting the driving force.
For example, when the medical device is an endoscopic treatment tool which is inserted for use into a channel of an endoscope, since an insertion section of the endoscope meanders inside a body, the insertion section of the medical device is constituted by a so-called flexible insertion section having flexibility with which it can be inserted into the meandering channel.
The transmission member is inserted into the insertion section of the medical device. When the insertion section is curved, the transmission member comes in contact with an inner wall of the insertion section, an inner wall of a transmission member sheath which is inserted into the insertion section and into which the transmission member is inserted, or the like and causes friction with the contact object. Because of this friction, a phenomenon in which a force acting on a distal end of the transmission member is smaller than a force applied to a proximal end and a desired driving force is not sufficiently transmitted occurs.
Regarding this problem, Patent Literature 1 proposes use of a wire which is formed by joining two types of wires having different rigidities by welding or the like. By disposing the wire having the higher rigidity on the proximal end side, a large force reflecting attenuation due to friction can be applied to generate a desired driving force at the distal end.

[Citation List]

[Patent Literature]

[Patent Literature 1]
Japanese Patent No. 5080702

[Summary of Invention]

[Technical Problem]

The technique described in Patent Literature 1 is excellent but still has the following problem.
That is, in the technique described in Patent Literature 1, since two types of wires are arranged in the longitudinal direction and are joined by welding or the like, stress is likely to be concentrated on the joint portion. As a result, even when the rigidity of the wire itself is sufficient, the wire may be fractured at the joint portion.
In consideration of the above-mentioned technique, an object of the present invention is to provide a medical wire that can appropriately generate a desired driving force at a distal end in a medical device having a flexible insertion section.
Another object of the present invention is to provide a medical device that has a flexible insertion section and can appropriately drive a mechanism at a distal end thereof.

[Solution to Problem]

According to a first aspect of the present invention, there is provided a medical wire including a main wire portion and a sub wire portion fixed to the main wire portion, wherein the main wire portion is entirely disposed in a longitudinal direction of the medical wire, and the medical wire has a first region in which a radial cross-sectional area is relatively small and a second region in which a radial cross-sectional area is larger than that of the first region.
According to a second aspect of the present invention, in the medical wire according to the first aspect, the sub wire portion may be disposed at only a part in a longitudinal direction of the medical wire.

According to a third aspect of the present invention, the medical wire according to the first or second aspect may further include a tapered portion that is disposed in a boundary portion between the first region and the second region and in which a radial dimension gradually decreases toward the first region.

According to a fourth aspect of the present invention, in the medical wire according to the first aspect, the second region may be disposed in both end portions of the medical wire in the longitudinal direction thereof.

According to a fifth aspect of the present invention, there is provided a medical device including: a long insertion section that has flexibility; a treatment section that is disposed at a distal end of the insertion section and has an end effector; an operation section that is disposed at the insertion section and configured to operate the treatment section; and the medical wire according to any one of the above-mentioned aspects, wherein the first region is connected to the treatment section and the second region is connected to the operation section.

According to a sixth aspect of the present invention, in the medical device according to the fifth aspect, the maximum tension which is generated in the second region by the operation section may be greater than the breaking strength of the first region and less than the breaking strength of the second region.

According to a seventh aspect of the present invention, the medical device according to the fifth aspect may further include a wire sheath that is inserted into the insertion section and into which the medical wire is inserted, and the wire sheath may have a small-diameter portion having a diameter smaller than that of other portions.

According to an eighth aspect of the present invention, in the medical device according to the fifth aspect, the treatment section may include a rigid support member that is disposed at the insertion section, and the medical wire may be disposed such that the first region is always located inside the support member.

According to a ninth aspect of the present invention, in the medical device according to the fifth aspect, the diameter of the treatment section may be smaller than the diameter of the insertion section.

According to a tenth aspect of the present invention, there is provided a medical device including: a long insertion section that has flexibility; a treatment section that is disposed at a distal end of the insertion section and has a rigid support member disposed at the insertion section and an end effector; an operation section that is disposed at the insertion section and configured to operate the treatment section; and the medical wire according to the fourth aspect, wherein the first region is disposed in a groove formed in the support member and connected to the treatment section and the second region is connected to the operation section.

[Advantageous Effects of Invention]

According to the medical wire of the present invention, it is possible to appropriately generate a desired driving force at the distal end even when the medical wire is applied to a medical device having a flexible insertion section.

According to the medical device of the present invention, it is possible to drive a mechanism at the distal end with a flexible insertion section.

[Brief Description of Drawings]

Fig. 1 is a side view showing a medical wire according to a first embodiment of the present invention.
Fig. 2 is a cross-sectional view taken along line I-I in Fig. 1.
Fig. 3 is a cross-sectional view taken along line II-II in Fig. 1.
Fig. 4 is a diagram showing a tapered portion in a modified example of the medical wire according to the first embodiment of the present invention.
Fig. 5 is a diagram showing a tapered portion in a modified example of the medical wire according to the first embodiment of the present invention.
Fig. 6 is a side view showing a medical wire according to a second embodiment of the present invention.
Fig. 7 is a partial cross-sectional view taken along line III-III in Fig. 6.
Fig. 8 is a cross-sectional view taken along line IV-IV in Fig. 6.
Fig. 9 is a schematic diagram showing a basic constitution of a medical device to which a medical wire according to the present invention is applied.
Fig. 10 is a diagram showing a first example of a connection aspect in which the medical wire according to the present invention is connected to the medical device.
Fig. 11 is a diagram showing a second example of the connection aspect in which the medical wire according to the present invention is connected to the medical device.
Fig. 12 is a diagram showing the second example of the connection aspect in which the medical wire according to the present invention is connected to the medical device.

Fig. 13 is a diagram showing a third example of the connection aspect in which the medical wire according to the present invention is connected to the medical device.

Fig. 14 is a diagram showing another aspect of the third example.

Fig. 15 is a diagram showing a fourth example of the connection aspect in which the medical wire according to the present invention is connected to the medical device.

Fig. 16 is a diagram showing another example of a treatment section in the fourth example.

Fig. 17 is a diagram showing another example of a treatment section in the fourth example.

Fig. 18 is a diagram showing an example in which the medical wire is connected to a curving section in the fourth example.

Fig. 19 is a diagram showing a modified example of the medical wire which is used in the fourth example.

Fig. 20 is a cross-sectional view showing a support member in which the medical wire according to the modified example shown in Fig. 19 is disposed.

Fig. 21 is a cross-sectional view showing another example of the support member in which the medical wire according to the modified example shown in Fig. 19 is disposed.

Fig. 22 is a diagram showing an example of the entire constitution of a medical manipulator to which the medical wire according to the present invention can be applied.

Fig. 23 is a cross-sectional view of a second region in a modified example of the medical wire according to the present invention.

Fig. 24 is a cross-sectional view of the second region in a modified example of the medical wire according to the present invention.

Fig. 25 is a cross-sectional view of the second region in a modified example of the medical wire according to the present invention.

Fig. 26 is a cross-sectional view showing a modified example of the medical wire according to the present invention.

[Description of Embodiments]

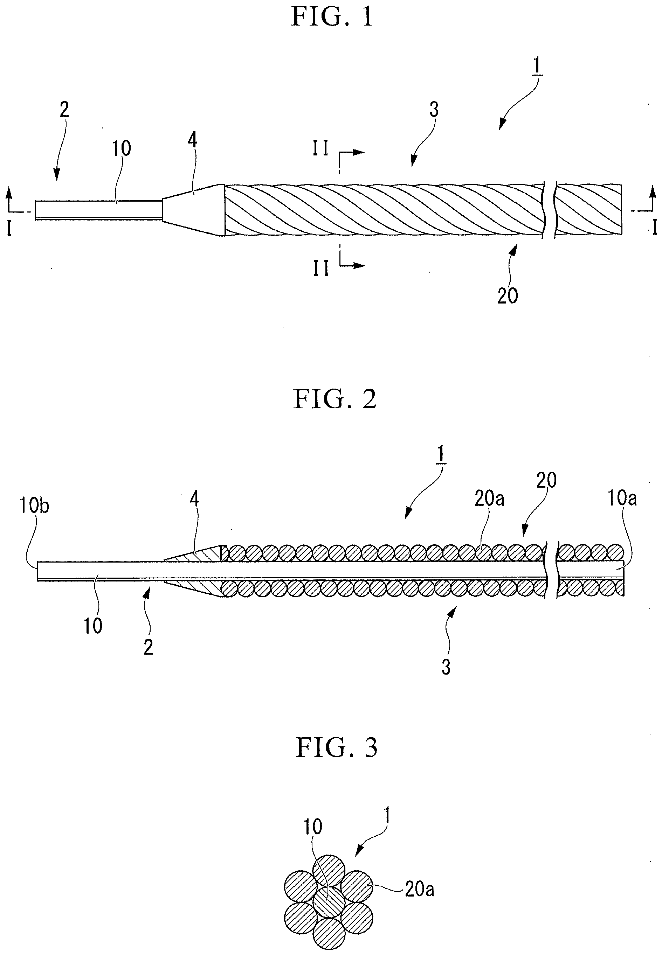

A first embodiment of the present invention will be described below with reference to Figs. 1 to 5.

Fig. 1 is a side view of a medical wire 1 according to this embodiment. The medical wire 1 includes a main wire portion 10 that is entirely disposed in a longitudinal direction and a sub wire portion 20 that is attached to a part of the main wire portion 10 in the longitudinal direction thereof.

Fig. 2 is a cross-sectional view taken along line I-I in Fig. 1, and Fig. 3 is a cross-sectional view taken along line II-II in Fig. 1. The sub wire portion 20 is disposed to cover the outer circumferential surface of the main wire portion over a predetermined length from one end 10a of the main wire portion 10. In the following description, the one end 10a is referred to as a proximal end 10a, and the opposite end 10b at which the sub wire portion is not disposed is referred to as a distal end 10b.

As shown in Fig. 3, the sub wire portion 20 includes a plurality of unit wires 20a. The plurality of unit wires 20a are arranged in the circumferential direction of the main wire portion 10 and the plurality of unit wires 20a are twisted together to form the sub wire portion 20. This constitution can be obtained, for example, by removing strands of a wire formed by twisting a plurality of strands around the main wire over a predetermined length.

The main wire portion 10 and the unit wires 20a are formed of a metal such as stainless steel. The wire constituting the main wire portion 10 and the unit wires 20a may be single wires or stranded wires, and are all shown as single wires in the schematic diagrams of the present invention.

The sub wire portion 20 is joined to the main wire portion 10 by soldering, brazing, laser welding, or the like, and is fixed not to move relative to the main wire portion 10 in the longitudinal direction. The sub wire portion 20 only has to be joined to the main wire portion at least in the boundary between a first region and a second region to be described later, but may be joined to the main wire portion throughout the longitudinal direction thereof.

In the medical wire 1, a portion in which the sub wire portion 20 is disposed has a radial cross-sectional area larger than that of the portion in which only the main wire portion 10 is present. That is, the medical wire 1 includes a first region 2 in the distal end portion in which the radial cross-sectional area is relatively small and a second region 3 in the proximal end portion in which the radial cross-sectional area is relatively large. In the second region 3, the main wire portion 10 and the sub wire portion 20 are fixed not to move relative to each other in the longitudinal direction and move as a unified body.

The boundary portion between the first region 2 and the second region 3 is provided with a tapered portion 4 by soldering, brazing, laser welding, or the like, and the radial dimension thereof gradually and smoothly decreases from the second region 3 to the first region 2.

The operations and advantageous effects of the medical wire 1 having the above-mentioned constitution will be described below.

When the insertion section of the medical device is curved, a force acting on the distal end of the transmission member such as a wire due to friction or the like is smaller than the force applied from the proximal end as described above. In order to increase the force acting on the distal end, a larger force only has to be applied from the proximal end, but the upper limit of the force is equal to breaking tension of the wire. Accordingly, the upper limit of the force acting on the distal end is limited to the breaking tension.

In the medical wire 1 according to this embodiment, since the second region 3 in the proximal end portion has a radial cross-sectional area greater than that of the first region 2, it is possible to apply a larger force than the breaking tension of the main wire portion 10 from the proximal end by appropriately setting the radial cross-sectional area of the second region 3. That is, the maximum value of the tension which can be generated in the second region 3 is larger than the breaking tension of the first region 2 and smaller than the breaking tension of the second region 3. As a result, even when the insertion section of the medical device is curved, a larger force than that in a normal wire in which the radial cross-sectional area is constant over the entire length can be made to act on the distal end portion. Since the second region 3 has greater rigidity with an increase in the radial cross-sectional area thereof, the second region is less likely to be elongated in the longitudinal direction than the first region 2. Since the elongation in the longitudinal direction of the transmission member in addition to the above-mentioned friction contributes to attenuation of the force in the distal end portion, the medical wire 1 can prevent the attenuation of the force in this aspect and a satisfactorily large force can be applied to the distal end portion.

In the medical wire 1, unlike the wire described in Patent Literature 1, the main wire portion 10 and the sub wire portion 20 come in contact with each other throughout the longitudinal direction of the sub wire portion 20. Accordingly, the contact area of the main wire portion 10 and the sub wire portion 20 can be made to be large. As a result, occurrence of a state in which the main wire portion 10 and the sub wire portion 20 are disjoined due to a large friction force therebetween can be significantly reduced. In the study of the inventors of the present invention, a wire having the same structure as the medical wire 1 and a wire in which the wire forming the first region and the wire forming the second region were joined only at ends in the longitudinal direction were compared in breaking strength in a state in which the radial cross-sectional areas of the first region and the second region were set to be equal to each other, and the result was that the breaking strength of the former was five times the breaking strength of the latter or more. The main wire portion was broken in the former wire, but breakage was caused in the latter wire due to disjoining of the joint portions before the main wire portion was broken.

The tapered portion 4 is disposed in the boundary portion between the first region 2 and the second region 3. Accordingly, even when the medical wire 1 is inserted into a buckling-prevention wire sheath (to be described later) or the like, the unit wire 20a in the vicinity of the boundary portion is not likely to be hooked to the wire sheath or the like and the medical wire can be satisfactorily driven forward and backward.

In this embodiment, the tapered portion is not essential. Accordingly, the main wire portion and the sub wire portion may be joined to each other by, for example, caulking or the like using a member and the boundary portion between the first region and the second region may be configured to have the largest radial dimension.

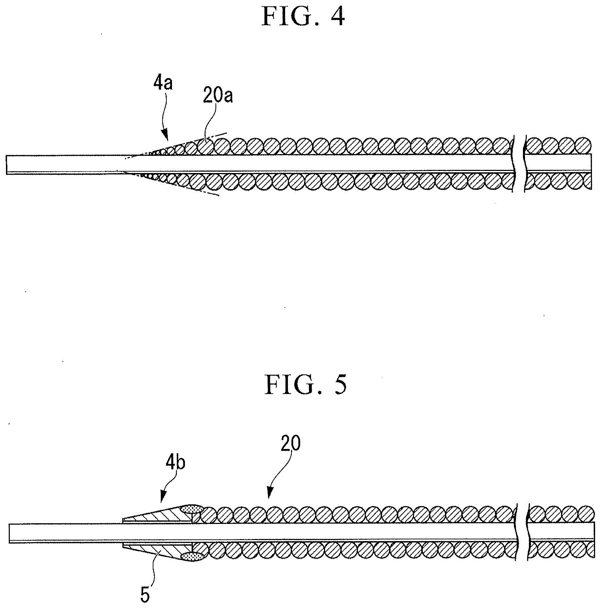

When the tapered portion is formed, the forming method thereof is not limited to the above-mentioned aspect. For example, as in a modified example shown in Fig. 4, a tapered portion 4a may be formed by gradually decreasing the thickness of the plurality of unit wires 20a toward the distal end through a known swaging process or the like. As in a modified example shown in Fig. 5, a tapered portion 4b may be formed by covering the distal end portion of the sub wire portion 20 with a cover 5 having substantially a truncated cone shape and joining the cover 5 to the sub wire portion 20 by soldering, brazing, laser welding, or the like.

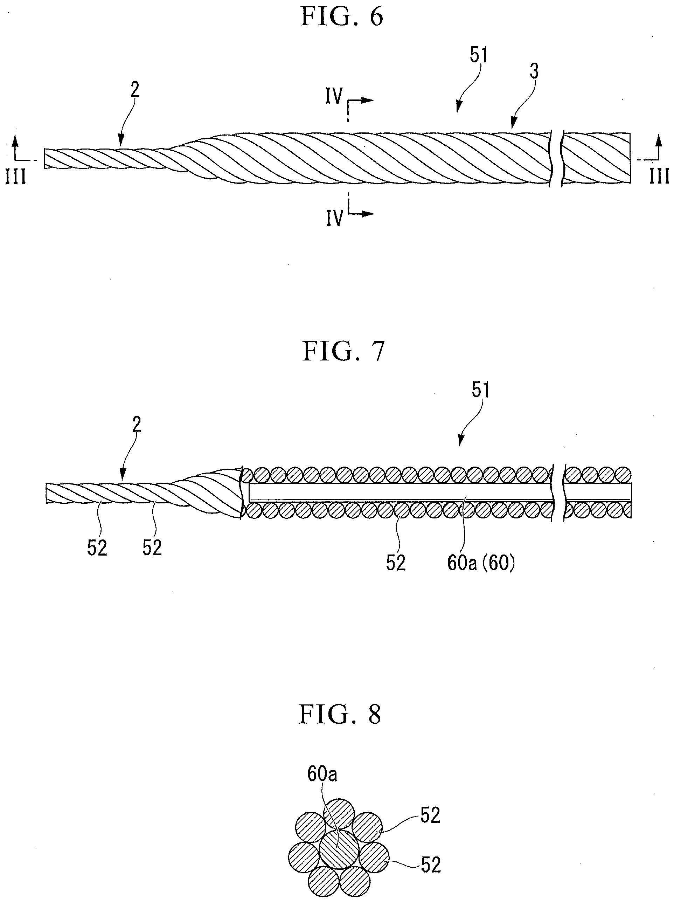

A second embodiment of the present invention will be described below with reference to Figs. 6 to 8. This embodiment is different from the first embodiment in constitutions of the main wire portion and the sub wire portion. In the following description, constitutions and the like common to those mentioned above will be referenced by the same reference numerals and description thereof will not be repeated.

Fig. 6 is a side view schematically showing a medical wire 51 according to this embodiment. Fig. 7 is a partial cross-sectional view taken along line III-III in Fig. 6, and Fig. 8 is a cross-sectional view taken along line IV-IV in Fig. 6.

As shown in Figs. 6 to 8, a sub wire portion 60 of the medical wire 51 is constituted by a single unit wire 60a. A main wire portion 52 is constituted by seven wires which are arranged in the circumferential direction of the unit wire 60a. The main wire portion 52 is disposed entirely in the longitudinal direction of the medical wire 51, and the first region 2 is formed by twisting the seven wires constituting the main wire portion 52 together.

In the medical wire 51 according to this embodiment, similarly to the medical wire 1 according to the first embodiment, a large force can be made to act on the distal end portion even when the insertion section of the medical device is curved.

Since the seven wires constituting the main wire portion 52 are arranged around the sub wire portion 60 and only these wires are twisted together to form the first region 2, the boundary portion between the first region and the second region is formed in a taper shape without performing a particular process of forming a tapered portion or the like. Accordingly, it is possible to realize a structure in which the wire is not likely to be hooked to the wire sheath or the like.

In the medical wire 51 according to the present invention, the sub wire portion 60 may be disposed throughout the longitudinal direction of the medical wire. With this configuration, it is possible to realize a structure having the first region and the second region, for example, by decreasing the diameter of only a part disposed in the first region in at least one of the main wire portion and the sub wire portion.

A third embodiment of the present invention will be described below. In this embodiment, a medical device having the medical wire according to the present invention will be described. The above-mentioned advantageous effects can be obtained by simply applying the medical wire according to the present invention to the medical device, but it may be possible to construct a medical device exhibiting an additional advantageous effect by studying the arrangement or the like. Various constitutional examples of the medical device will be described below.

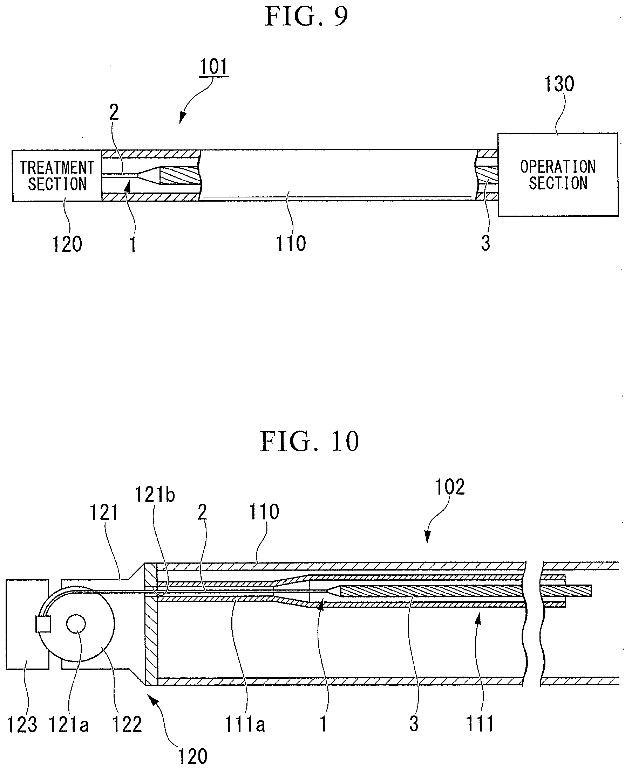

Fig. 9 shows a basic constitution of a medical device to which the present invention is suitably applied. The medical device 101 includes a long insertion section 110 having flexibility, a treatment section 120 disposed at a distal end of the insertion section 110, and an operation section 130 disposed at a proximal end of the insertion section 110.

The insertion section 110 has a known constitution having a sheath formed of a resin or a coil, a metal hose, and the like. As will be described later, the insertion section may have a curving section that can be actively curved in a part thereof.

The treatment section 120 has an end effector that is disposed at the distal end of the medical device 101 to exhibit a certain effect. Examples of the end effector include various surgical devices such as a grasping forceps or a high-frequency knife or an observation device such as an imaging unit having an imaging device and a light source.

The operation section 130 serves to drive the medical wire 1 inserted into the insertion section 110 forward and backward in the longitudinal direction of the insertion section 110. The medical wire 1 may be manually driven by an operator or the like or may be electrically driven by a motor or the like. One of various known constitutions can be appropriately selected and employed as the operation section 130 depending on whether driving is manual or electrical.

The medical wire 1 is inserted into the insertion section 110. The first region 2 in the distal end portion of the medical wire 1 is connected to an end effector or a member for driving the end effector in the treatment section 120. The second region 3 in the proximal end portion of the medical wire 1 is connected to the operation section 130. Accordingly, by driving the medical wire 1 forward and backward via the operation section 130, the treatment section 120 can be driven to exhibit a desired effect.

In the medical device 101, in view of sufficient exhibition of the advantageous effects of the medical wire 1, it is preferable that tension applied to the second region 3 from the operation section 130 be set to be equal to or greater than the breaking strength of the first region 2 and less than the breaking strength of the second region 3. Accordingly, a large force can be made to act on the first region 2. The magnitude of the force to be applied to the first region 2 can be appropriately set to be in a range less than the breaking strength of the first region 2. The magnitude of the tension which needs to be applied to the second region 3 based on the magnitude to be applied to the first region 2 can be calculated by computation. For example, when the tension in the first region 2 is defined as $T_0$, the friction coefficient with the member coming into contact with the medical wire is defined as $\mu$, and the curving angle of the insertion section 110 is defined as $\theta$, the tension T which needs to be applied to the second region 3 can be calculated as in Equation (1). In Equation (1), e denotes a natural logarithm.

$$T = T_0 e^{\mu\theta} \qquad \qquad \ldots(1)$$

Various aspects for insertion of the medical wire 1 into the insertion section 110 and connection of the first region to the end effector or the like can be considered.

In a medical device 102 according to a first example shown in Fig. 10, the treatment section 120 includes a rigid support member 121 that has a rotation shaft 121 a, a pulley 122 that is rotatably supported by the rotation shaft 121a, and an end effector 123 that is attached to the pulley 122. The support member 121 is attached to the distal end of the insertion section 110 and an end of the first region 2 of the medical wire 1 is fixed to the pulley 122.

The medical wire 1 is inserted into a buckling-prevention wire sheath 111 that is inserted into the insertion section 110. The distal end of the wire sheath 111 is fixed to the support member 121, and a hole 121 b formed in the support member 121 communicates with the wire sheath 111. The distal end portion of the wire sheath 111 forms a small-diameter portion 111a having a diameter smaller than that of the proximal end portion. The inner diameter of the small-diameter portion 111 a is set to a size into which the second region 3 of the medical wire 1 cannot be inserted.

In the medical device 102 according to the first example, since the distal end portion of the wire sheath 111 can be decreased in diameter due to the small-diameter portion 111 a, it is possible to decrease the dimension of the support member 121. Since the second region 3 does not need to be inserted into the treatment section, the pulley 122 or the end effector 123 can be decreased in size and the treatment section can be decreased to be smaller in diameter than the insertion section as shown in Fig. 10. When the medical device 102 is an endoscopic treatment tool that is inserted into a channel of an endoscope, or the like, the area of the treatment section 120 in the visual field of the endoscope can be decreased and thus treatment can be easily performed while appropriately checking surrounding tissues. The minimum diameter of the pulley around which the wire is wound is limited according to the diameter of the wire. However, in the medical wire 1, since the radial dimension of the first region 2 is small, it is possible to appropriately achieve a decrease in size of the pulley.

Since the clearance between the first region 2 and the inner surface of the wire sheath 111 can be decreased by forming the small-diameter portion 111a, the medical wire 1 is not likely to move in the radial direction in the wire sheath 111 (not likely to rattle). Accordingly, it is possible to control the medical wire 1 to smoothly move forward and backward. By forming the small-diameter portion 111a, there is also an advantageous effect that it is possible to improve curvedness of the region in which the small-diameter portion is formed in the insertion section.

In the first example, when the second region 3 comes in contact with the small-diameter portion 111a, the medical wire 1 cannot move forward any further. Accordingly, the length of the first region 2 and the length of the small-diameter portion 111a may be set in consideration of the maximum degree of movement of the end effector 123 (the maximum degree of rotation of the pulley 122). Alternatively, the second region 3 may be used as a stopper based on the fact that the medical wire cannot move forward.

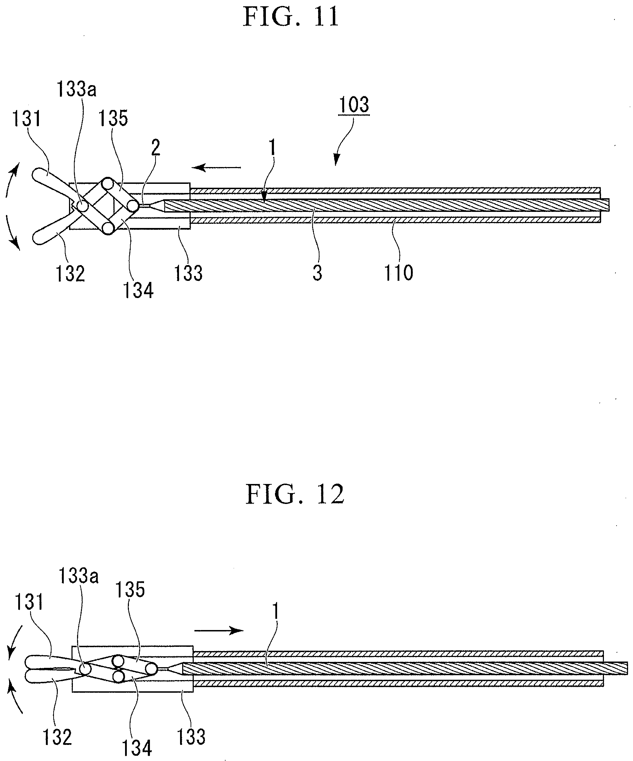

In a medical device 103 according to a second example shown in Figs. 11 and 12, a pair of forceps members 131 and 132 are provided as an end effector. The medical wire 1 is not inserted into the wire sheath but is inserted directly into the insertion section 110. The pair of forceps members 131 and 132 are rotatably supported by a rotation shaft 133a disposed in the support member 133. Link members 134 and 135 are connected to the proximal ends of the forceps members 131 and 132, and the proximal ends of the link members 134 and 135 are supported by the distal end portion of the medical wire 1. Accordingly, when the medical wire 1 is moved forward, the pair of forceps members 131 and 132 are opened as shown in Fig. 11. When the medical wire 1 is moved backward, the pair of forceps members 131 and 132 are closed as shown in Fig. 12.

When a wire is used as the transmission member, a force in the pulling direction can be easily transmitted but an operating force cannot be appropriately and easily transmitted in an operation in the inserting direction due to buckling or the like. Particularly, in the medical wire 1, the first region 2 has a smaller radial dimension and thus buckles more easily than the second region 3.

In the second example, in consideration of this point, the boundary between the first region 2 and the second region 3 is set to be located inside the support member 133 even when the medical wire 1 moves as far back as possible as shown in Fig. 12. Accordingly, since the first region 2 is always located inside the support member 133, the first region is not affected by curving of the insertion section 110 and it is thus possible to satisfactorily suppress occurrence of buckling or the like.

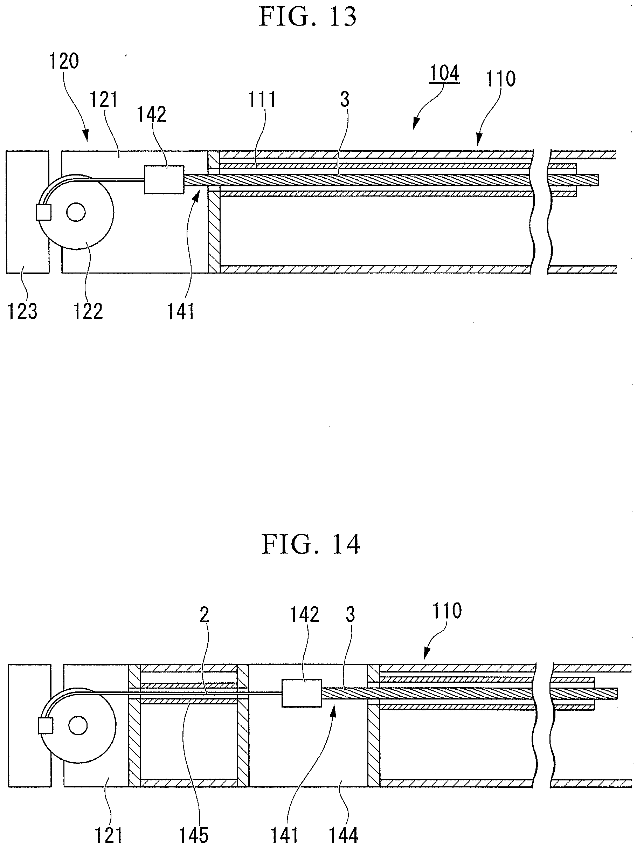

In a medical device 104 according to a third example shown in Figs. 13 and 14, a medical wire 141 in which a caulking member 142 is disposed at the boundary between the first region 2 and the second region 3 is used instead of the medical wire 1. The radial dimension of the medical wire 141 is the largest in the boundary portion between the first region 2 and the second region 3 due to the caulking member 142. Accordingly, when the medical wire 141 is inserted into the wire sheath 111, the inner diameter of the wire sheath 111 has to be set in consideration of the dimension of the caulking member 142.

Therefore, in the example shown in Fig. 13, the medical wire 141 is disposed such that the caulking member 142 is located inside the support member 121. In addition, when the caulking member 142 is set to be located inside the support member 121 even in a state in which the medical wire 141 moves as far back as possible, the caulking member 142 does not enter the wire sheath 111. Accordingly, the inner diameter of the wire sheath 111 can be set without considering the dimension of the caulking member 142. As a result, it is possible to achieve a decrease in diameter of the wire sheath 111 and to prevent rattling of the medical wire 141.

As shown in Fig. 14, even when a hard member 144 is disposed in the insertion section 110 separately from the support member 121 and the caulking member 142 is located in the range of the hard member 144, the same advantageous effects can be obtained. In this case, since a space for the caulking member does not have to be prepared in the support member, it is possible to decrease the sizes of the support member and the treatment section. When the hard member is disposed separately from the support member, the first region 2 between the hard member and the treatment section is likely to buckle. Accordingly, as shown in Fig. 14, it is preferable that an auxiliary wire sheath 145 be disposed between the support member 121 and the hard member 144. Since only the first region 2 passes through the auxiliary wire sheath 145, the dimension of the auxiliary wire sheath 145 can be set in consideration of only the first region 2.

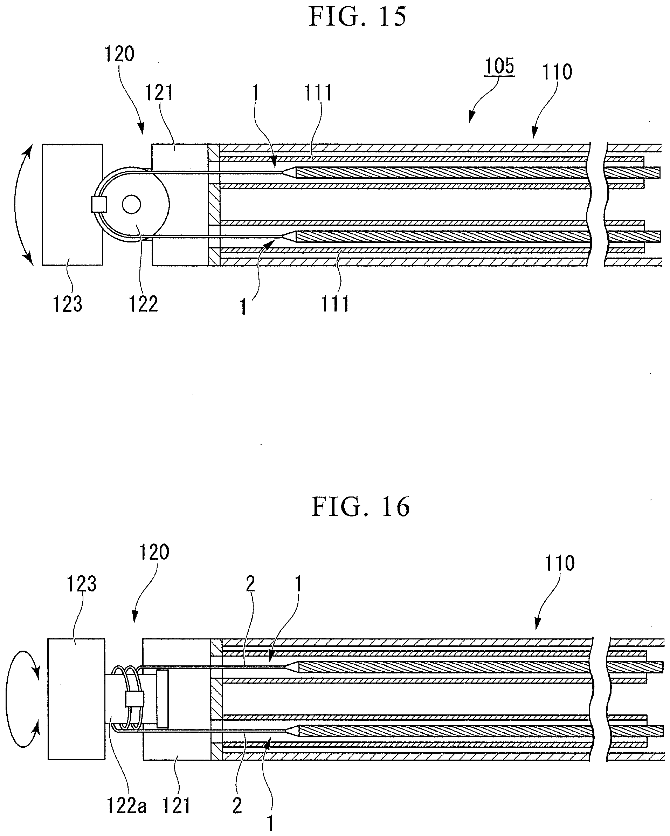

In a medical device 105 according to a fourth example shown in Fig. 15, two medical wires 1 are used. The distal ends of the medical wires 1 are fixed to the same pulley 122 and the pulley 122 can be made to rotate by pulling one medical wire 1. A structure in which the other medical wire is inserted when one medical wire is pulled may be employed if necessary.

When wires are used as the transmission member in so-called antagonistic drive using a pair of transmission members, it is known that the wires are elongated and loosened, and transmission of a force may not be satisfactory.

However, when the medical wire according to the present invention is used as the transmission member, even a small treatment section can be satisfactorily driven by the first region having a small radial dimension while transmitting a force using the second region which is not likely to be elongated.

The treatment section which is driven using a pair of medical wires is shown in Fig. 15, and various other constitutions may be used. In a treatment section 120 shown in Fig. 16, a pulley 122a is disposed to rotate about the axis of the insertion section 110 and the first regions 2 of the pair of medical wires 1 are wound around the pulley 122a and are fixed thereto. By inserting or pulling the pair of medical wires 1, the end effector 123 attached to the pulley 122a can be rotationally driven about the axis of the insertion section 110.

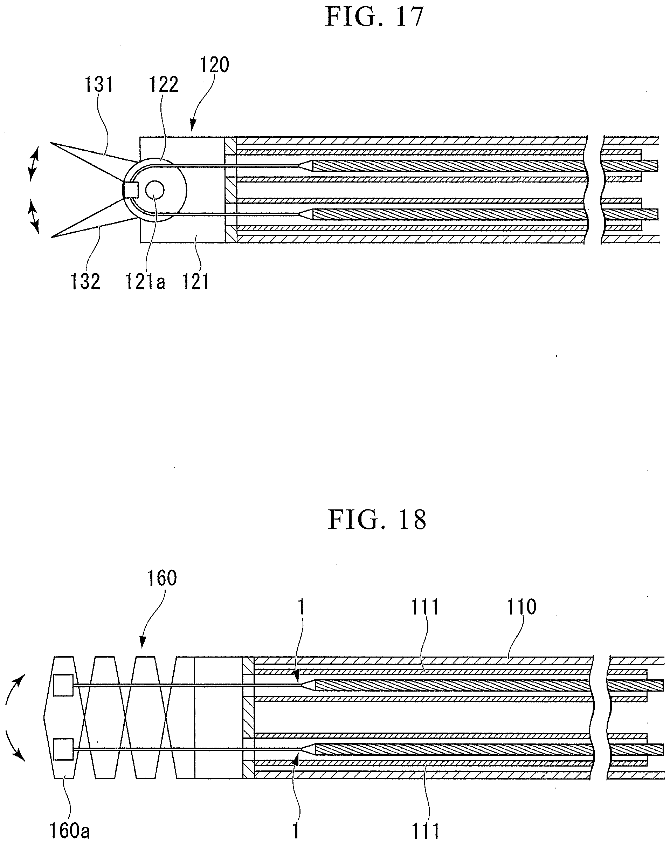

In a treatment section 120 shown in Fig. 17, one of a pair of forceps members 131 and 132 is attached to the pulley 122. A second pulley which is not shown is attached to be coaxial with the pulley 122 behind the pulley 122, and another pair of medical wires are fixed to the second pulley in the same way. The other of the pair of forceps members 131 and 132 is attached to the second pulley. In this example, by operating two pairs of medical wires to drive the pulley 122 and the second pulley in cooperation, the pair of forceps members 131 and 132 can be opened and closed and the direction of the forceps members in the closed state can also be changed.

The driving target of the medical wire 1 is not limited to the treatment section. In Fig. 18, a pair of medical wires 1 are connected to a curving section 160 for curving the insertion section 110. The curving section 160 has a known constitution in which a plurality of segment rings or curved segments (hereinafter referred to as "segment rings or the like") are arranged in the axial direction of the insertion section 110, and can curve the insertion section 110 in two directions by pulling and inserting the pair of medical wires connected to the segment ring or the like 160a at the most distal end.

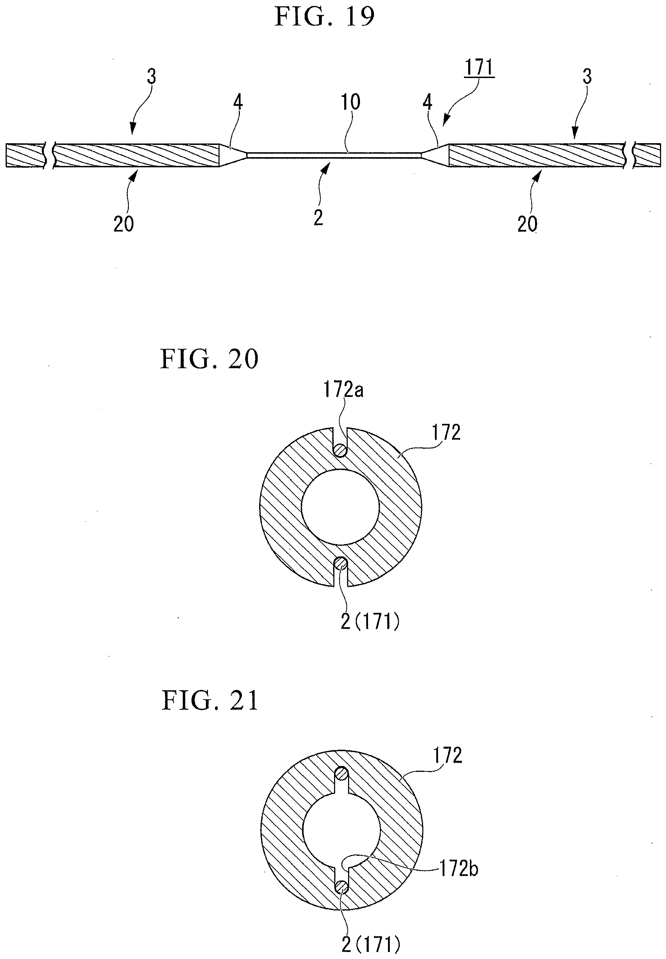

In the fourth example, a medical wire 171 having the second region 3 at both end portions in the longitudinal direction thereof may be used instead of the pair of medical wires 1 as shown in Fig. 19. Here, when a hole is formed in the support member and the second region 2 is inserted into the hole, the medical wire 171 cannot be inserted without setting the inner diameter of the hole to a value equal to or greater than the outer diameter of the second region 3. However, as shown in Fig. 20, by providing a support member 172 with a groove 172a having a width corresponding to the outer diameter of the first region 2, the first region 2 can be disposed in the treatment section. Since the second region does not have to be inserted into the groove 172a, the width of the groove 172a can be set to correspond to the diameter of the first region 2. The groove may be formed on the outer circumferential surface of the support member as shown in Fig. 20 or may be formed on the inner circumferential surface like a groove 172b shown in Fig. 21.

The medical device to which the medical wire according to the present invention is applied has been described above in conjunction with the first to fourth examples. These examples have particular advantageous effects and thus may be appropriately combined without departing from the gist thereof.

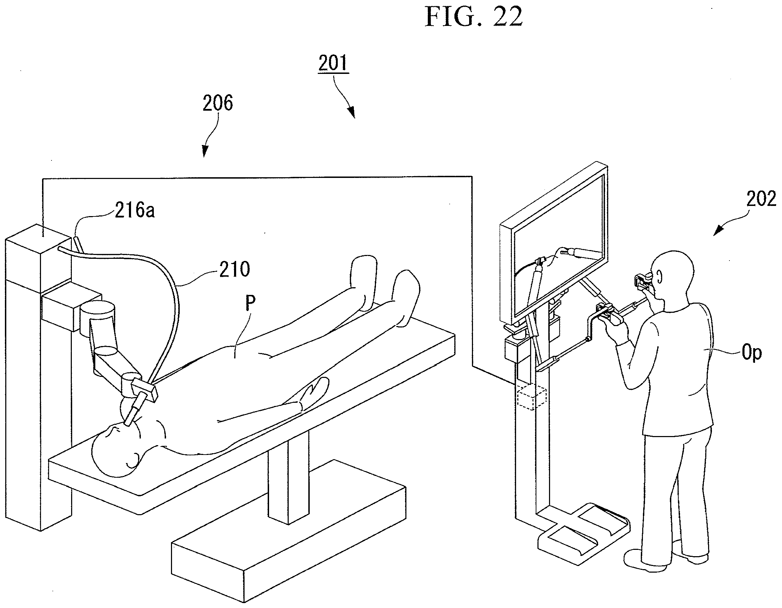

An example of the medical device according to the present invention includes a master-slave medical manipulator 201 as shown in Fig. 22. The medical manipulator 201 includes a master manipulator 202 which is operated by an operator Op and a slave manipulator 206 which is provided with a treatment endoscope device 210 which is inserted into a patient P, and the slave manipulator 206 operates with the operation of the master manipulator 202. In the medical manipulator 201, the medical wire according to the present invention may be used as a transmission member for driving an arm or the like disposed at the distal end of the treatment endoscope device 210, or may be used as a transmission member for driving a treatment tool which is inserted for use into a channel of the treatment endoscope device 210 from a feed port 216a.

While embodiments of the present invention have been described above, the technical scope of the present invention is not limited to the embodiments, but a combination of elements may be changed or the elements may be modified in various forms or be deleted without departing from the gist of the present invention. The present invention is not limited to the above-mentioned description.

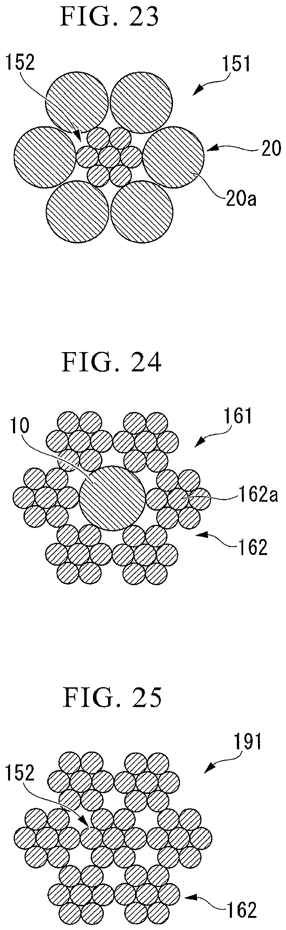

In constituting the medical wire according to the present invention, a single wire and a stranded wire may be combined for use as any one of the main wire portion and the sub wire portion. Accordingly, like a medical wire 151 according to a modified example shown in Fig. 23, a main wire portion 152 may be formed of a stranded wire and a sub wire portion 20 may be formed of a single unit wire 20a. Like a medical wire 161 according to a modified example shown in Fig. 24, the main wire portion 10 may be formed of a single wire and a sub wire portion 162 may be formed of a stranded unit wire 162a. In addition, like a medical wire 191 according to a modified example shown in Fig. 25, a structure including the main wire portion 152 and the sub wire portion 162 may be employed. Figs. 23 to 25 are cross-sectional views of the second region.

When a stranded wire is used, it is possible to improve flexibility of the medical wire in comparison with a case in which a single wire is used. For example, when a stranded wire is used for the first region, the first region can be more easily wound around a pulley having a smaller diameter. When a stranded wire is used for the second region, it is possible to reduce interference with a curving operation of the curving section.

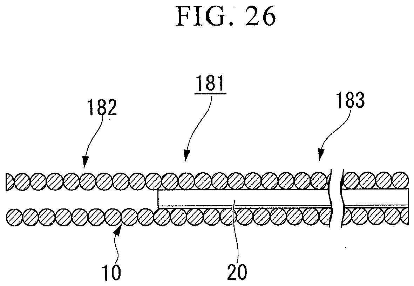

In the above-mentioned embodiments, the radial dimension of the first region is smaller than the radial dimension of the second region. However, as long as the radial cross-sectional area of the first region is smaller than the radial cross-sectional area of the second region, the first region 182 may be formed to be hollow like a medical wire 181 according to a modified example shown in Fig. 26. In this modified example, the radial dimensions of the first region 182 and the second region 183 are substantially equal to each other, but since the first region 182 is hollow, the radial cross-sectional area thereof is smaller than that of the second region 183. This medical wire does not contribute to a decrease in diameter of the treatment section, but can appropriately generate a desired driving force at the distal end in a medical device having a flexible insertion section, similarly to the medical wires according to the above-mentioned embodiments.

In the above-mentioned embodiments, at least a part of the sub wire portion is joined to the main wire portion, but the joint portion may not be provided as long as the main wire portion and the sub wire portion are satisfactorily fixed not to move relative to each other due to a frictional force or the like therebetween.

[Industrial Applicability]

According to the medical wire, it is possible to provide a medical wire that can appropriately generate a desired driving force at the distal end in a medical device having a flexible insertion section. According to the medical device, it is possible to provide a medical device that has a flexible insertion section and can appropriately drive a tool at the distal end thereof.

[Reference Signs List]

| | |
|---|---|
| 1, 51, 141, 151, 161, 171, 181, 191 | Medical wire |
| 2, 182 | First region |
| 3, 183 | Second region |
| 4, 4a, 4b | Tapered portion |
| 10, 52, 152 | Main wire portion |
| 20, 60, 162 | Sub wire portion |
| 101, 102, 103, 104, 105 | Medical device |
| 110 | Insertion section |
| 120 | Treatment section |
| 130 | Operation section |

**Claims**

1. A medical wire comprising a main wire portion and a sub wire portion fixed to the main wire portion, wherein the main wire portion is entirely disposed in a longitudinal direction of the medical wire, and the medical wire has a first region in which a radial cross-sectional area is relatively small and a second region in which a radial cross-sectional area is larger than that of the first region.

2. The medical wire according to claim 1, wherein the sub wire portion is disposed at only a part of the medical wire in the longitudinal direction thereof.

3. The medical wire according to claim 1 or 2, further comprising a tapered portion that is disposed in a boundary portion between the first region and the second region and in which a radial dimension gradually decreases toward the first region.

4. The medical wire according to claim 1, wherein the second region is disposed in both end portions of the medical wire in the longitudinal direction thereof.

5. A medical device comprising:

   a long insertion section that has flexibility;
   a treatment section that is disposed at a distal end of the insertion section and has an end effector;
   an operation section that is disposed at the insertion section and configured to operate the treatment section; and
   the medical wire according to any one of claims 1 to 4,
   wherein the first region is connected to the treatment section and the second region is connected to the operation section.

6. The medical device according to claim 5, wherein a maximum value of tension which is generated in the second region by the operation section is greater than breaking strength of the first region and less than breaking strength of the second region.

7. The medical device according to claim 5, further comprising a wire sheath that is inserted into the insertion section and into which the medical wire is inserted,
   wherein the wire sheath has a small-diameter portion having a diameter smaller than that of other portions.

8. The medical device according to claim 5, wherein the treatment section includes a rigid support member that is disposed at the insertion section, and
   the medical wire is disposed such that the first region is always located inside the support member.

9. The medical device according to claim 5, wherein the diameter of the treatment section is smaller than the diameter of the insertion section.

10. A medical device comprising:

    a long insertion section that has flexibility;
    a treatment section that is disposed at a distal end of the insertion section and has a rigid support member disposed at the insertion section, and an end effector;
    an operation section that is disposed at the insertion section and configured to operate the treatment section; and
    the medical wire according to claim 4,
    wherein the first region is disposed in a groove formed in the support member and connected to the treatment section and the second region is connected to the operation section.

# FIG. 1

# FIG. 2

# FIG. 3

FIG. 4

FIG. 5

# FIG. 6

# FIG. 7

# FIG. 8

## FIG. 9

## FIG. 10

# FIG. 11

# FIG. 12

# FIG. 13

# FIG. 14

## FIG. 15

## FIG. 16

## FIG. 17

## FIG. 18

FIG. 19

FIG. 20

FIG. 21

# FIG. 22

EP 3 100 666 A1

FIG. 23

FIG. 24

FIG. 25

# FIG. 26

**INTERNATIONAL SEARCH REPORT**

International application No.

PCT/JP2015/050942

A.   CLASSIFICATION OF SUBJECT MATTER
*A61B1/00*(2006.01)i,  *A61B17/28*(2006.01)i

According to International Patent Classification (IPC) or to both national classification and IPC

B.   FIELDS SEARCHED

Minimum documentation searched (classification system followed by classification symbols)
A61B1/00-1/32, A61B17/28

Documentation searched other than minimum documentation to the extent that such documents are included in the fields searched

| | | | |
|---|---|---|---|
| Jitsuyo Shinan Koho | 1922–1996 | Jitsuyo Shinan Toroku Koho | 1996–2015 |
| Kokai Jitsuyo Shinan Koho | 1971–2015 | Toroku Jitsuyo Shinan Koho | 1994–2015 |

Electronic data base consulted during the international search (name of data base and, where practicable, search terms used)

C.   DOCUMENTS CONSIDERED TO BE RELEVANT

| Category* | Citation of document, with indication, where appropriate, of the relevant passages | Relevant to claim No. |
|---|---|---|
| X<br>Y<br>A | JP 61-141341 A  (Olympus Optical Co., Ltd.),<br>28 June 1986 (28.06.1986),<br>page 2, upper right column, line 5 to page 3,<br>lower left column, the last line; fig. 1 to 3<br>(Family: none) | 1-3<br>4-7,9,10<br>8 |
| X | JP 8-131441 A  (Olympus Optical Co., Ltd.),<br>28 May 1996 (28.05.1996),<br>paragraphs [0075] to [0077]; fig. 20<br>(Family: none) | 1,2 |
| X | JP 2004-041319 A  (Olympus Corp.),<br>12 February 2004 (12.02.2004),<br>paragraphs [0001], [0021] to [0024], [0033] to<br>[0043]; fig. 4, 8, 9<br>(Family: none) | 1,2 |

☒   Further documents are listed in the continuation of Box C.       ☐   See patent family annex.

| | | |
|---|---|---|
| * | Special categories of cited documents: | |
| "A" | document defining the general state of the art which is not considered    to be of particular relevance | "T"   later document published after the international filing date or priority date and not in conflict with the application but cited to understand the principle or theory underlying the invention |
| "E" | earlier application or patent but published on or after the international filing date | "X"   document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&"   document member of the same patent family |

| Date of the actual completion of the international search | Date of mailing of the international search report |
|---|---|
| 06 April 2015 (06.04.15) | 14 April 2015 (14.04.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | |
| | Telephone No. |

Form PCT/ISA/210 (second sheet) (July 2009)

| | INTERNATIONAL SEARCH REPORT | International application No. |
| | | PCT/JP2015/050942 |

**C (Continuation).** DOCUMENTS CONSIDERED TO BE RELEVANT

| Category* | Citation of document, with indication, where appropriate, of the relevant passages | Relevant to claim No. |
|---|---|---|
| Y | JP 2013-111160 A  (Fujifilm Corp.),<br>10 June 2013 (10.06.2013),<br>paragraphs [0047] to [0051]; fig. 4<br>& US 2013/0137928 A1    & CN 103126645 A | 4,10 |
| Y | JP 8-224247 A  (Olympus Optical Co., Ltd.),<br>03 September 1996 (03.09.1996),<br>paragraphs [0034], [0035]; fig. 4, 5<br>(Family: none) | 5-7,9 |
| A | JP 2009-233225 A  (Fujinon Corp.),<br>15 October 2009 (15.10.2009),<br>paragraphs [0013] to [0016]; fig. 1, 2<br>(Family: none) | 1-10 |

Form PCT/ISA/210 (continuation of second sheet) (July 2009)

**EP 3 100 666 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014014398 A

- JP 5080702 B